# FASCICULE DE BREVET EUROPEEN

(11) **EP 0 475 857 B1**
(45) Date de publication et mention de la délivrance du brevet: **22.03.1995**
(21) Numéro de dépôt: 91420293.2
(22) Date de dépôt: 14.08.1991
(51) Int. Cl.: A61M 5/32

(54) **Dispositif à aiguille à usage médical**
Nadelvorrichtung für medizinische Zwecke
Needle assembly for medical use

(30) Priorité: 03.09.1990 FR 9011093
(43) Date de publication de la demande: 18.03.1992
(73) Titulaire: HOSPAL INDUSTRIE, F-69883 Meyzieu Cédex (FR)
(72) Inventeur: Chevallet, Jacques, F-69360 Serezin Du Rhone (FR); Santiago, Yvon, F-69800 Saint-Priest (FR)

(56) Documents cités:
- EP-A- 0 363 180
- AU-B- 451 181
- US-A- 3 595 230
- US-A- 4 681 567
- US-A- 4 723 943
- US-A- 4 941 881

## Description

La présente invention concerne un dispositif à aiguille à usage médical comprenant une aiguille reliée à un tube souple et un fourreau pour enclore l'aiguille.

Ce type de dispositif est utilisé dans divers secteurs d'activité médicale, notamment pour transfuser, perfuser ou relier un patient au circuit sang d'un rein artificiel. Dans le premier cas, le tube souple est connecté à une poche destinée à recueillir le sang du donneur ; dans les autres cas, l'extrémité libre du tube souple est munie d'un élément de connection permettant le raccordement du dispositif à l'extrémité, munie d'un élément de connection complémentaire, d'une ligne de perfusion ou d'une ligne artérielle ou veineuse du circuit sang du rein artificiel.

Après utilisation, un dispositif à aiguille du type mentionné plus haut présente un danger potentiel car il a été au contact du sang d'un patient pouvant être contaminé par divers germes pathogènes (virus de l'hépatite ou du sida, en particulier) et l'aiguille, de par sa forme prévue pour traverser les tissus, est particulièrement propre à infliger des blessures et, par là, à inoculer ces germes.

Il est donc prescrit aux utilisateurs de ne pas réutiliser ces dispositifs et, préalablement à leur mise au rebut, de mettre en place sur l'aiguille un organe de protection en rendant la pointe inaccessible. L'organe de protection généralement proposé à cette fin est le capuchon dans lequel l'aiguille est enclose pour en protéger la pointe en fin de fabrication du dispositif à aiguille, avant stérilisation. Cet organe de protection, pour efficace qu'il soit une fois en place, présente toutefois cet inconvénient majeur que sa mise en place elle-même n'est pas sans risque : elle nécessite en effet que l'utilisateur rapproche de sa main avec laquelle il tient le capuchon l'aiguille dont il oriente la pointe vers l'ouverture du capuchon, c'est-à-dire aussi en direction de sa main et l'on comprend que, dans un moment de fatigue, d'inattention ou d'énervement, des accidents puissent se produire. Un autre inconvénient de cet organe de protection est qu'il peut être enlevé.

Pour pallier les insuffisances de ce mode de protection, le brevet US 4 676 783 propose un organe de protection distinct du capuchon placé sur l'aiguille avant stérilisation du dispositif à aiguille. Cet organe de protection est constitué par un fourreau ouvert à ses deux extrémités, concentrique au tube souple. Le fourreau est prévu pour coulisser sur le tube entre deux positions délimitées par une butée annulaire formée sur un élément de raccord de l'aiguille au tube souple, qui est toujours à l'intérieur du fourreau : une première position où l'extrémité du fourreau située du côté de l'aiguille est arrêtée par la butée et où l'aiguille est totalement découverte, le dispositif à aiguille étant alors en position d'utilisation ; une deuxième position où l'autre extrémité du fourreau est arrêtée par la butée, l'aiguille étant alors complètement enclose dans le fourreau. Le fourreau comprend un ergot faisant saillie dans son canal intérieur pour s'engager sur la butée annulaire dans la deuxième position du fourreau et bloquer le fourreau par rapport à l'aiguille.

Cet organe de protection présente certains inconvénients, particulièrement sensibles dans les cas, nombreux, d'utilisation du dispositif à aiguille où l'aiguille doit être enfoncée dans une fistule ou un vaisseau sous-cutané. Dans ces cas, l'aiguille doit être mise en place le plus tangentiellement possible à la peau. Or le fourreau décrit ci-dessus, dans la mesure où il présente un certain encombrement, étant concentrique au tube et où, en position d'utilisation du dispositif, il s'étend sur le tube à partir de la base de l'aiguille, impose un angle d'incidence non négligeable de l'aiguille par rapport à la peau. En outre, ce fourreau interdit l'utilisation d'un organe de préhension du type a ailes de papillon pour faciliter la mise en place et le retrait de l'aiguille dans un vaisseau, ce type d'organe de préhension étant fixé à la base de l'aiguille sur l'élément de raccord au tube souple. Enfin, dans la mesure où sa mise en place sur l'aiguille est irréversible, ce fourreau ne peut être utilisé pour enclore l'aiguille qu'après qu'elle a servi.

Le brevet US 4 941 881 décrit un dispositif à aiguille à usage médical comprenant :
- une aiguille reliée par sa base à un tube ;
- un organe de préhension à ailes de papillon s'étendant latéralement à partir de la base de l'aiguille ;
- un fourreau ouvert à ses extrémités et concentrique au tube pour découvrir ou enclore l'aiguille, ce fourreau comportant une fente s'étendant sur une partie de sa longueur pour permettre le passage de l'organe de préhension lors du déplacement du fourreau sur l'aiguille ; et
- des moyens de blocage pour immobiliser l'aiguille à l'intérieur du fourreau de façon irréversible après usage.

Ce dispositif à aiguille comprend donc un fourreau pour enclore l'aiguille qui est compatible avec l'utilisation d'un organe de préhension à ailes de papillon, qui n'a pas d'incidence sur l'angle de pénétration de l'aiguille dans un vaisseau sous-cutané et qui n'expose pas l'utilisateur au risque de se piquer lors de sa mise en place sur l'aiguille. Ce dispositif nécessite cependant un capuchon pour protéger l'aiguille avant usage, dont la manipulation n'est pas exempte de tout risque pour l'utilisateur.

L'invention a pour objet un dispositif à aiguille du type mentionné ci-dessus comprenant un fourreau pour enclore l'aiguille qui permette de faire l'économie d'un capuchon de protection de l'aiguille et qui soit sans risque pour l'utilisateur.

Selon l'invention, on prévoit un dispositif à aiguille à usage médical comprenant :
- une aiguille reliée par sa base à un tube, un élément saillant s'étendant latéralement à partir de la base de l'aiguille ;
- un fourreau ouvert à ses extrémités et concentrique au tube pour découvrir on enclore l'aiguille, ce fourreau comportant au moins une première fente s'étendant sur au moins une partie de sa longueur pour permettre le passage de l'élément saillant lors du déplacement du fourreau sur l'aiguille ;
- des premiers moyens de blocage pour immobiliser l'aiguille à l'intérieur du fourreau de façon irréversible après usage, caractérisé en ce qu'il comprend des seconds moyens de blocage pour immobiliser l'aiguille à l'intérieur du fourreau de façon réversible pour la protéger avant usage.

Selon une caractéristique de l'invention, les seconds moyens de blocage sont constitués par l'élément saillant et une ouverture correspondante dans le fourreau située sur la fente, le dispositif comprenant des moyens pour faciliter l'introduction de l'élément saillant dans la fente à partir de l'ouverture.

Selon une autre caractéristique de l'invention, les premiers moyens de blocage sont constitués par l'élément saillant et une ouverture correspondante dans le fourreau communiquant avec la fente.

Selon un premier mode de réalisation de l'invention, le dispositif à aiguille comprend une seule fente s'étendant sur au moins une partie de la longueur du fourreau.

L'ouverture des premiers moyens de blocage est située, par exemple, sur la fente, l'ouverture des seconds moyens de blocage étant située entre l'ouverture des premiers moyens de blocage et l'extrémité du fourreau située du côté de l'aiguille. Selon une variante, l'ouverture des premiers moyens de blocage est située latéralement par rapport à l'ouverture des seconds moyens de blocage avec laquelle elle communique par un passage, le dispositif comprenant des moyens pour faciliter l'introduction de l'élément saillant dans l'ouverture des premiers moyens de blocage à partir de l'ouverture des seconds moyens de blocage.

Selon un deuxième mode de réalisation de l'invention, le dispositif à aiguille comprend une première et une seconde fentes s'étendant sur une partie de la longueur du fourreau. Les premiers moyens de blocage comprennent alors, par exemple, l'élément saillant et une ouverture correspondante dans le fourreau située dans le prolongement la première fente et les seconds moyens de blocage comprennent l'élément saillant et une ouverture correspondante dans le fourreau située dans le prolongement de la seconde fente, le dispositif comprenant des moyens pour faciliter l'introduction de l'élément saillant dans la première fente à l'extrémité de la fente située du côté de l'aiguille et des moyens pour faciliter l'introduction de l'élément saillant dans la seconde fente à partir de l'ouverture des seconds moyens de blocage.

D'autres caractéristiques et avantages de la présente invention apparaîtront à la lecture de la description suivante, faite en relation aux dessins annexés sur lesquels :
- La figure 1, a, b, c, est une vue en perspective d'un premier mode de réalisation de l'invention, dans trois positions d'utilisation différentes ;
- La figure 2 est une vue en perspective d'un deuxième mode de réalisation de l'invention ;
- la figure 3, a, b, c, est une vue en perspective d'un troisième mode de réalisation de l'invention dans trois positions d'utilisation différentes ; et
- La figure 4, a, b, est une vue en perspective d'un quatrième mode de réalisation de l'invention, dans deux positions d'utilisation différentes.

Dans la suite, on désignera les parties identiques des différents mode de réalisation de l'invention décrits par les mêmes chiffres de référence.

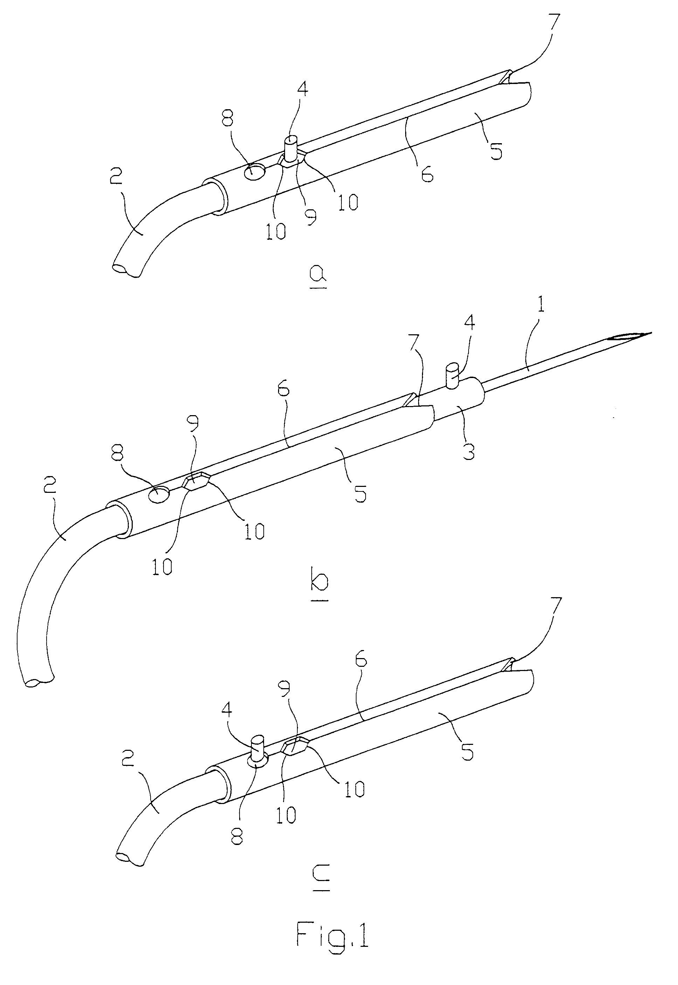

Le dispositif à aiguille à usage médical représenté sur la figure 1 comprend une aiguille 1 reliée par sa base à un tube souple 2 au moyen d'un élément de raccord 3. Un téton 4, dont la fonction principale sera explicitée plus loin, fait saillie sur l'élément de raccord 3, perpendiculairement à l'axe de l'aiguille 1. Accessoirement le téton 4 sert à repérer la position du biseau de l'aiguille 1. Un fourreau tubulaire 5 est passé sur le tube 2 où il peut coulisser librement. Le fourreau 5 est fendu sur une partie de sa longueur à partir de son extrémité située du côté de l'aiguille 1 (qui sera appelée extrémité avant, dans la suite).

La fente 6 du fourreau 5, dont les bords sont jointifs sur la majeure partie de sa longueur, est élargie par une découpe 7 en forme de V à l'extrémité avant du fourreau, pour guider le téton 4 et faciliter sa pénétration dans la fente 6. Le fourreau 5 comprend en outre une ouverture 8 située dans le prolongement de la fente 6, de dimension au moins égale à la section transversale du téton 4. La distance séparant l'ouverture 8 de l'extrémité avant du fourreau 5 est supérieure à la longueur de l'aiguille 1. On note que, contrairement à ce qu'on observe à son autre extrémité, la fente 6 n'est pas élargie au niveau de sa jonction à l'ouverture 8. Le fourreau est réalisé dans un matériau ayant une élasticité suffisante pour s'ouvrir élastiquement lorsque le téton 4 est forcé dans la fente 6.

Le fourreau 5 comporte une seconde ouverture 9 située sur la fente 6, à proximité de l'ouverture 8, la distance séparant la seconde ouverture 9 de l'extrémité avant du fourreau 5 étant supérieure à la longueur de l'aiguille 1. A la différence de ce qu'on observe sur la première ouverture 8, la fente 6 est élargie par une découpe en V 10 au niveau de ses deux points de jonction à la seconde ouverture 9, pour favoriser la pénétration du téton 4 dans la fente 6 en direction de la première ouverture 8 comme en direction de l'extrémité avant du fourreau 5.

L'intérêt de ce fourreau est qu'il est utilisable pour protéger l'aiguille 1 avant l'utilisation du dispositif à aiguille. En effet, en fin de fabrication du dispositif à aiguille, le fourreau 5 est passé sur le tube souple 2 et est emmanché sur l'aiguille 1 jusqu'à ce que le téton 4 parvienne dans la seconde ouverture 9 (figure 1a). Le dispositif à aiguille est alors conditionné et stérilisé. Pour mettre le dispositif en position d'utilisation (figure 1b), il suffit à l'utilisateur de prendre le fourreau 5 d'une main, de caler le téton 4 de son autre main, et de tirer sur le fourreau pour engager le téton 4 dans la fente 6 et l'y faire glisser jusqu'à ce qu'il échappe. Après usage du dispositif, l'utilisateur fait l'opération inverse pour replacer l'aiguille 1 à l'intérieur du fourreau 5, mais il engage le fourreau au maximum sur l'aiguille 1 de façon que le téton 5 vienne se loger dans la première ouverture 8 (figure 1c). L'aiguille 1 est alors irréversiblement bloquée dans le fourreau 5 et le dispositif ne fait courir aucun risque au personnel chargé de sa mise au rebut.

Il est important de noter que pendant les manipulations qui viennent d'être décrites, les deux mains de l'utilisateur restent en arrière de l'aiguille 1.

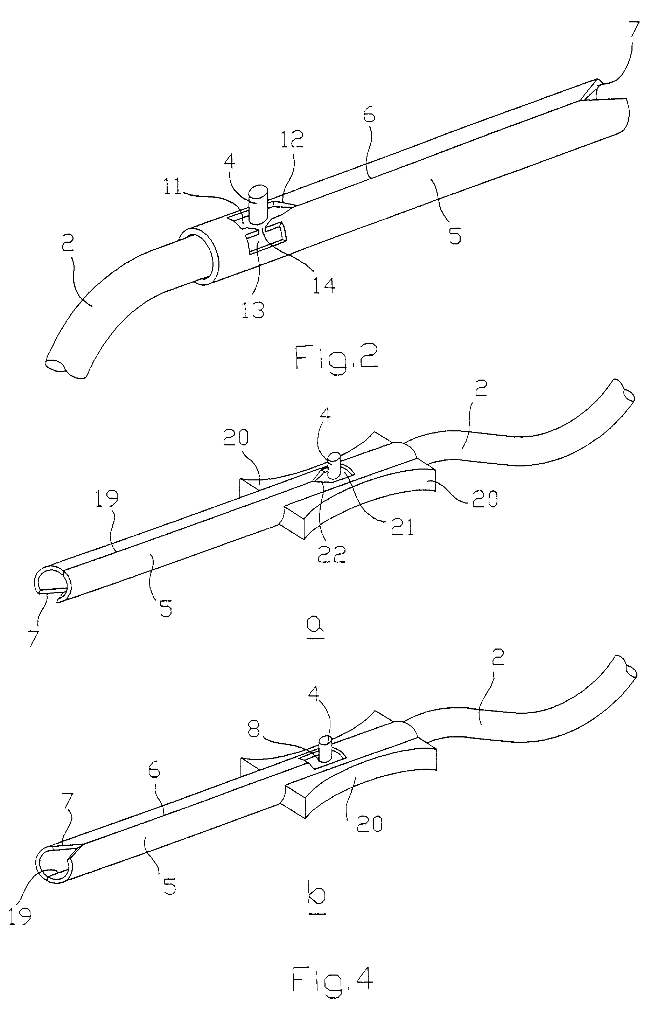

La figure 2 représente un dispositif à aiguille dont le fourreau 5, comme le précédent, est prévu pour être utilisé pour protéger l'aiguille 1 avant l'utilisation du dispositif. Ce fourreau comprend une ouverture 11 située dans le prolongement de la fente 6, à laquelle la fente 6 se raccorde en s'élargissant (découpe en V 12). La distance séparant l'ouverture 11 de l'extrémité avant du fourreau 5 est supérieure à la longueur de l'aiguille 1. L'ouverture 11 communique avec une seconde ouverture 13 latérale, par l'intermédiaire d'un passage 14 allant en se rétrécissant de l'ouverture 11 à l'ouverture 13 de façon à faciliter le passage du téton 4 dans un sens et à l'entraver en sens inverse.

En fin de fabrication, ce dispositif à aiguille se présente comme illustré sur la figure 2. Pour mettre le dispositif en position d'utilisation, l'utilisateur tire le fourreau 5 d'une main en direction du tube souple 2, tout en maintenant le téton 4 de l'autre main. Après usage, l'utilisateur pratique en sens inverse pour ramener le téton 4 dans la première ouverture 11. Après quoi, il fait pivoter le fourreau 5 par rapport à l'ensemble aiguille-tube souple pour forcer le téton 4 dans le passage 14 et le faire pénétrer dans la seconde ouverture 13. Etant donné la forme du passage 14, l'opération inverse sera impossible à réaliser, au moins involontairement.

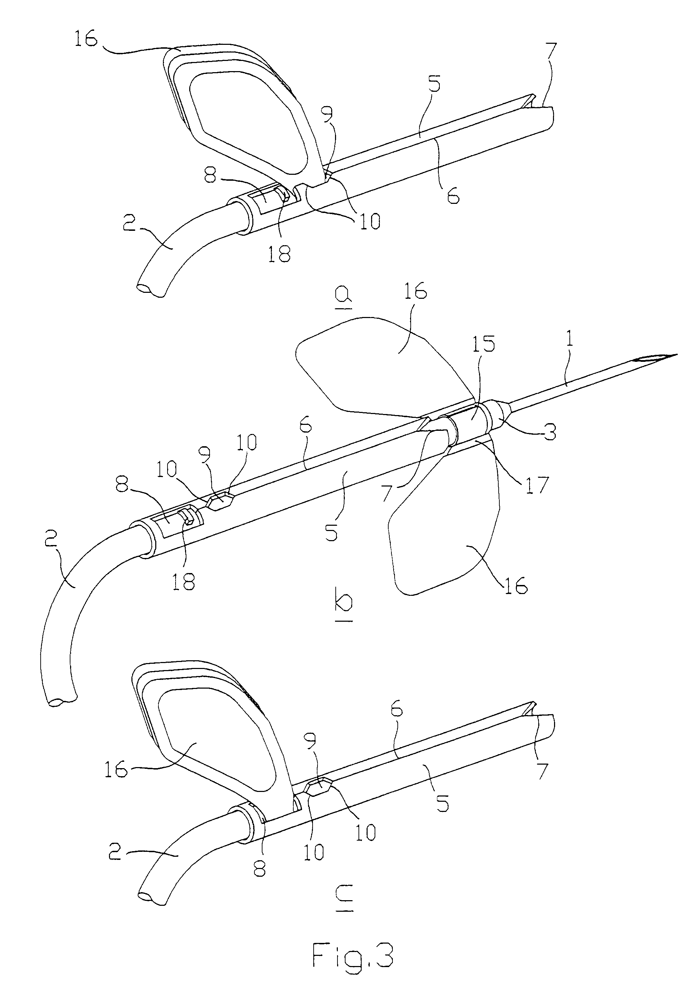

La figure 3 représente un dispositif à aiguille qui se différencie essentiellement de ceux qui ont été décrits précédemment en ce qu'il est muni d'un organe de préhension du type à ailes de papillon, d'un type connu en soi, qui est destiné à faciliter l'implantation et le retrait de l'aiguille 1 dans une fistule ou un vaisseau sous-cutané, et à fournir un élément de stabilisation pour le dispositif une fois mis en place sur un patient. Cet organe de préhension comprend un corps tubulaire 15 fixé sur l'élément de raccord 3 de l'aiguille 1 au tube souple 2, corps auquel sont articulées deux ailes 16 par l'intemédiaire de charnières 17. Les ailes 16 peuvent être repliées l'une contre l'autre (position de préhension illustrée sur les figures 3a et 3c) et elles peuvent être aussi déployées totalement (position de stabilisation illustrée sur la figure 3b : un morceau de sparadrap fixant les ailes ainsi déployées au bras du patient empêche l'aiguille 1 de pivoter par rapport à son axe d'implantation initial).

Dans ce mode de réalisation, ce sont les ailes 16 repliées l'une contre l'autre qui jouent le rôle d'élément saillant (dévolu dans les dispositifs des figures 1 et 2 au téton 4) destiné à coopérer avec les ouvertures du fourreau 5 pour immobiliser l'aiguille 1 à l'intérieur du fourreau 5, de façon réversible ou irréversible selon le type d'ouverture.

Le fourreau 5 comprend une première ouverture 8 située dans le prolongement de la fente 6, ayant des dimensions suffisantes pour laisser le passage à la base des ailes 16. Les bords de la fente 6 sont jointifs au niveau de sa jonction à l'ouverture 8. Le fourreau 5 comprend en outre une seconde ouverture 9, située sur la fente 6 à proximité de l'ouverture 8, la fente 6 s'élargissant au niveau de ses deux points de jonction à l'ouverture 9 (découpes en V 10). La distance séparant l'ouverture 9 de l'extrémité avant du fourreau 5 est supérieure à la longueur de l'aiguille. On remarque que l'ouverture 8 est barrée par une butée transversale 18 ayant une zone de moindre résistance au niveau de son raccordement au fourreau 5. La butée 18 a pour fonction d'empêcher les ailettes 16 de pénétrer complètement dans l'ouverture 8.

Au sortir de fabrication, ce dispostif à aiguille se présente comme illustré sur la figure 3a : le fourreau 5 est emmanché sur l'aiguille 1, qu'il enclot complètement, l'arrière des ailes 16 étant au contact de la butée 18 et les ailes elles mêmes étant pincées par les bords de la fente 6 s'étendant entre les deux ouvertures 8, 9. Dans cette position, l'aiguille 1 peut être sortie du fourreau 5 pour être mise en place sur un patient. Il suffit à l'utilisateur de saisir les ailes 14 d'une main et de tirer le fourreau 5 de l'autre en direction du tube souple 2, la fente 6 élargie au niveau de sa jonction à l'ouverture 9 permettant aux ailes de pénétrer dans la fente 6 en en écartant les bords. Le dispositif est alors dans sa position d'utilisation illustrée sur la figure 3b.

Pour retirer le dispositif mis en place sur un patient, l'utilisateur commence par faire sauter la butée 18 en la faisant pivoter vers l'extérieur autour de sa zone fragilisée.

Il saisit ensuite les ailes 16 en les repliant l'une contre l'autre et il retire l'aiguille 1 du vaisseau ou de la fistule du patient. Il fait alors coulisser de sa main libre le fourreau 5 en direction de l'aiguille 1, fait pénétrer les ailes dans la fente 6 par la découpe 7 et les fait glisser jusqu'à ce qu'elles aient pénétré dans l'ouverture 8, d'où il est impossible de les faire ressortir par un mouvement inverse.

On note qu'avec ce mode de réalisation de l'invention aussi, les deux mains de l'utilisateur restent en arrière de l'aiguille au cours des manipulations de retrait et de mise en place du fourreau de/sur l'aiguille 1.

Le dispositif à aiguille représenté sur la figure 4 se différencie de ceux qui ont été décrits plus haut en ce que son fourreau 5 comprend non pas une mais deux fentes 6, 19 diamétralement opposées s'étendant à partir de l'extrémité avant du fourreau 5. Le fourreau 5 est muni de deux éléments de renfort 20 s'étendant à partir de son extrémité arrière pour maintenir rapprochés étroitement les deux moitiés de fourreau définies par les fentes 6, 19. Les éléments de renfort 20 sont profilés pour faciliter la préhension du fourreau 5 entre le pouce et l'index. Les fentes 6, 19 sont respectivement prolongées par deux ouvertures 8, 21 dimentionnées pour recevoir le téton 4. La distance séparant les ouvertures 8, 21 de l'extrémité avant du fourreau 5 est supérieure à la longueur de l'aiguille 1. Une découpe en V 7 élargit la fente 6 à l'extrémité avant du fourreau 5, tandis que les bords de la fente 6 sont jointifs au niveau de sa jonction à l'ouverture 8. Inversement, les bords de la fente 19 sont jointifs à l'extrémité avant du fourreau 5 tandis qu'une découpe en V 22 élargit la fente 19 au niveau de sa jonction à l'ouverture 21.

En fin de fabrication, avant stérilisation, l'aiguille 1 est enclose dans le fourreau 5 dans la position représentée sur la figure 4a, le téton 4 passant par l'ouverture 21 et bloquant l'aiguille 1 dans le fourreau 5. L'aiguille 1 peut cependant être sortie du fourreau 5 grâce à la découpe en V 22 qui favorise l'introduction du téton 4 dans la fente 19 lorsqu'on tire le fourreau 5 en direction du tube 2 tout en maintenant fixe le téton 4, traction qui aboutit à faire échapper le téton 4 de la fente 19.

Après usage, la seule façon possible de replacer l'aiguille 1 dans le fourreau 5 consite à faire pivoter le fourreau de 180° par rapport à la position qui occupe sur la figure 4a, de façon à présenter le téton 4 dans la découpe en V7. En tirant le téton 5 en direction de l'arrière du fourreau 5 tout en maintenant le fourreau 5 fixe, on provoque l'introduction du téton 4 dans la fente 6 et la rentrée de l'aiguille 1 dans le fourreau 5 jusqu'à ce que le téton 4 pénètre dans l'ouverture 8 (figure 4b). Le mouvement inverse n'est pas possible, les bords jointifs de la fente 6 au niveau de sa jonction à l'ouverture 8 s'opposant à l'introduction du téton 4 dans la fente. Comme les dispositifs à aiguille des figures 2, 3, 4 ce dispositif comporte donc aussi deux types de moyens de blocage pour immobiliser l'aiguille 1 dans le fourreau 5 respectivement de façon réversible avant usage (téton 4, ouverture 21) et de façon irréversible après usage (téton 4, ouverture 8).

La présente invention n'est pas limitée aux modes de réalisation qui viennent d'être décrits et elle est susceptible de variantes qui apparaîtront à l'homme de l'art. Ainsi, les moyens pour favoriser l'introduction de l'élément saillant (téton 4, ailes 16) dans la fente 6, 17, qui sont constitués dans les exemples décrits par les découpes en V 7, 10, 22 élargissant la fente 6 à ses extrémités, pourraient être remplacés par des moyens équivalents : les bords de la fente pourraient ainsi présenter un écartement constant sur toute leur longueur et l'élément de blocage saillant être muni de bords d'attaque effilés (téton 4 de section transversale triangulaire, par exemple). En outre le fourreau 5 pourrait être fendu sur toute sa longueur et la fente n'être pas rectiligne mais hélicoïdale.

## Revendications

1. Dispositif à aiguille à usage médical comprenant :
- une aiguille (1) reliée par sa base à un tube (2), un élément saillant (4 ; 16) s'étendant latéralement à partir de la base de l'aiguille (1),
- un fourreau (5) ouvert à ses extrémités et concentrique au tube (2) pour découvrir ou enclore l'aiguille (1), ce fourreau (5) comportant au moins une première fente (6) s'étendant sur au moins une partie de sa longueur pour permettre le passage de l'élément saillant (4 ; 16) lors du déplacement du fourreau (5) sur l'aiguille (1),
- des premiers moyens de blocage (4, 16 ; 8, 13) pour immobiliser l'aiguille (1) à l'intérieur du fourreau (5) de façon irréversible après usage,
caractérisé en ce qu'il comprend des seconds moyens de blocage (4, 16 ; 9, 11, 21) pour immobiliser l'aiguille (1) à l'intérieur du fourreau (5) de façon réversible pour la protéger avant usage.

2. Dispositif à aiguille selon la revendication 1, caractérisé en ce que les seconds moyens de blocage sont constitués par l'élément saillant (4 ; 16) et une ouverture (9 ; 11) correspondante dans le fourreau (5) située sur la fente (6), le dispositif comprenant des moyens (10 ; 12) pour faciliter l'introduction de l'élément saillant (4 ; 16) dans la fente (6) à partir de l'ouverture (9 ; 11).

3. Dispositif à aiguille selon une des revendications 1 et 2, caractérisé en ce que les premiers moyens de blocage sont constitués par l'élément saillant (4 ; 16) et une ouverture (9 ; 11) correspondante dans le fourreau (5) communiquant avec la fente (6)

4. Dispositif à aiguille selon les revendications 2 et 3, caractérisé en ce que l'ouverture (8) des premiers moyens de blocage est située sur la fente (6) et en ce que l'ouverture (9) des seconds moyens de blocage est située entre l'ouverture (8) des premiers moyens de blocage et l'extrémité du fourreau (5) située du côté de l'aiguille (1).

5. Dispositif à aiguille selon les revendication 2 et 3, caractérisé en ce que l'ouverture (13) des premiers moyens de blocage est située latéralement par rapport à l'ouverture (11) des seconds moyens de blocage avec laquelle elle communique par un passage (12), le dispositif comprenant des moyens pour faciliter l'introduction de l'élément saillant (4 ; 16) dans l'ouverture (13) des premiers moyens de blocage à partir de l'ouverture (11) des seconds moyens de blocage.

6. Dispositif à aiguille selon la revendication 1, caractérisé en ce qu'il comprend une seconde (19) fente s'étendant sur une partie de la longueur du fourreau (5) et en ce que les seconds moyens de blocage comprennent l'élément saillant (4) et une ouverture (21) correspondante dans le fourreau (5) située dans le prolongement de la seconde fente (19), le dispositif comprenant des moyens (20) pour faciliter l'introduction de l'élément saillant (4 ; 16) dans la seconde fente (19) à partir de l'ouverture (21) des seconds moyens de blocage.

7. Dispositif à aiguille selon une des revendications 1 à 6, caractérisé en ce qu'il comprend des moyens (7) pour faciliter l'introduction de l'élément saillant (4 ; 16) dans la première fente (6), à l'extrémité de la fente (6) située du côté de l'aiguille (1).

8. Dispositif selon une des revendications 6 et 7, caractérisé en ce que les moyens pour faciliter l'introduction de l'élément saillant (4 ; 16) dans la fente (6 ; 19) sont constitués par une découpe en V (7 ; 10 ; 22) élargissant les bords de la fente (6 ; 19).

9. Dispositif selon une des revendications 1 à 8, caractérisé en ce que l'élément saillant est constitué par les ailes (16) repliées l'une contre l'autre d'un organe de préhension du type à ailes de papillon (15, 16, 17) solidaire d'un élément de raccord (3) reliant la base de l'aiguille (1) au tube (2).

## Claims

1. Needle assembly for medical use, comprising:
- a needle (1) connected via its base to a tube (2), a protruding element (4; 16) extending laterally from the base of the needle (1),
- a sleeve (5) open at its ends and concentric to the tube (2) for exposing or enclosing the needle (1), this sleeve (5) comprising at least a first slit (6) extending over at least a part of its length in order to permit the passage of the protruding element (4; 16) during the displacement of the sleeve (5) on the needle (1),
- first blocking means (4, 16; 8, 13) for immobilizing the needle (1) inside the sleeve (5) in an irreversible manner after use,
characterized in that it comprises second blocking means (4, 16; 9, 11, 21) for immobilizing the needle (1) inside the sleeve (5) in a reversible manner in order to protect it before use.

2. Needle assembly according to Claim 1, characterized in that the second blocking means are formed by the protruding element (4; 16) and a corresponding opening (9; 11) situated on the slit (6) in the sleeve (5), the assembly comprising means (10; 12) for facilitating the introduction of the protruding element (4; 16) into the slit (6) starting from the opening (9; 11).

3. Needle assembly according to either of Claims 1 and 2, characterized in that the first blocking means are formed by the protruding element (4; 16) and a corresponding opening (9; 11) in the sleeve (5) communicating with the slit (6).

4. Needle assembly according to Claims 2 and 3, characterized in that the opening (8) of the first blocking means is situated on the slit (6), and in that the opening (9) of the second blocking means is situated between the opening (8) of the first blocking means and that end of the sleeve (5) situated towards the needle (1).

5. Needle assembly according to Claims 2 and 3, characterized in that the opening (13) of the first blocking means is situated laterally in relation to the opening (11) of the second blocking means, with which opening it communicates via a passage (12), the assembly comprising means for facilitating the introduction of the protruding element (4; 16) into the opening (13) of the first blocking means starting from the opening (11) of the second blocking means.

6. Needle assembly according to Claim 1, characterized in that it comprises a second slit (19) extending over a part of the length of the sleeve (5), and in that the second blocking means comprise the protruding element (4) and a corresponding opening (21) situated in the sleeve (5) in the continuation of the second slit (19), the assembly comprising means (20) for facilitating the introduction of the protruding element (4; 16) into the second slit (19) starting from the opening (21) of the second blocking means.

7. Needle assembly according to one of Claims 1 to 6, characterized in that it comprises means (7) for facilitating the introduction of the protruding element (4; 16) into the first slit (6), at that end of the slit (6) situated towards the needle (1).

8. Assembly according to either of Claims 6 and 7, characterized in that the means for facilitating the introduction of the protruding element (4; 16) into the slit (6; 19) are formed by a V-shaped cutout (7; 10; 22) widening the edges of the slit (6; 19).

9. Assembly according to one of Claims 1 to 8, characterized in that the protruding element is formed by the wings (16), folded one against the other, of a gripping element of the type with butterfly wings (15, 16, 17) and integral with a connection element (3) joining the base of the needle (1) to the tube (2).

## Patentansprüche

1. Nadelvorrichtung zur medizinischen Verwendung mit:
- einer Nadel (1), die durch ihre Basis mit einem Rohr (2) verbunden ist, wobei ein vorspringendes Element (4; 16) sich ausgehend von der Basis der Nadel (1) seitlich erstreckt,
- einer Buchse (5), die an ihren Enden offen und konzentrisch zum Rohr (2) ist, um die Nadel (1) zu bedecken oder zu umschließen, wobei die Buchse (5) zumindest einen ersten Schlitz (6) umfaßt, der sich zumindest über einen Teil seiner Länge erstreckt, um den Durchtritt des vorspringenden Elements (4; 16) bei einer Verstellung der Buchse (5) auf der Nadel (1) zu erlauben;
- ersten Blockiermitteln (4, 16; 8, 13), um die Nadel (1) im Innern der Buchse (5) in nicht umkehrbarer Weise nach der Verwendung unbeweglich zu machen,
dadurch gekennzeichnet, daß sie zweite Blockiermittel (4, 16; 9, 11, 21) zum Unbeweglichmachen der Nadel (1) im Innern der Buchse (5) in umkehrbarer Weise umfaßt, um sie vor der Verwendung zu schützen.

2. Nadelvorrichtung nach Anspruch 1, dadurch gekennzeichnet, daß die zweiten Blockiermittel durch das vorspringendes Element (4; 16) und eine entsprechende Öffnung (9; 11) in der Buchse (5) gebildet sind, die auf dem Schlitz (6) angeordnet ist, wobei die Vorrichtung Mittel (10; 12) zum Erleichtern der Einführung des vorspringenden Elements (4; 16) in den Schlitz (6) ausgehend von der Öffnung (9; 11) umfaßt.

3. Nadelvorrichtung nach Anspruch 1 oder 2, dadurch gekennzeichnet, daß die ersten Blockiermittel durch das vorspringendes Element (4; 16) und eine entsprechende Öffnung (9; 11) in der Buchse (5) gebildet sind, die mit dem Schlitz (6) in Verbindung steht.

4. Nadelvorrichtung nach Anspruch 2 oder 3, dadurch gekennzeichnet, daß die Öffnung (8) der ersten Blockiermittel auf dem Schlitz (6) angeordnet sind, und daß die Öffnung (9) der zweiten Blockiermittel zwischen der Öffnung (8) der ersten Blockiermittel und dem Ende der Buchse (5) angeordnet ist, die auf der Seite der Nadel (1) angeordnet ist.

5. Nadelvorrichtung nach Anspruch 2 oder 3, dadurch gekennzeichnet, daß die Öffnung (13) der ersten Blockiermittel seitlich in bezug auf die Öffnung (11) der zweiten Blockiermittel angeordnet ist, mit der sie über einen Durchgang (12) in Verbindung steht, wobei die Vorrichtung Mittel zum Erleichtern der Einführung des vorspringenden Elements (4; 16) in die Öffnung (13) der ersten Blockiermittel ausgehend von der Öffnung (11) der zweiten Blockiermittel umfaßt.

6. Nadelvorrichtung nach Anspruch 1, dadurch gekennzeichnet, daß sie einen zweiten Schlitz (19) umfaßt, der sich über einen Teil der Länge der Buchse (5) erstreckt, und daß die zweiten Blockiermittel das vorspringende Element (4) und eine entsprechende Öffnung (21) in der Buchse (5) umfassen, die in der Verlängerung des zweiten Schlitzes (19) angeordnet ist, wobei die Vorrichtung Mittel (20) zum Erleichtern der Einführung des vorspringenden Elements (4; 16) in den zweiten Schlitz (19) ausgehend von der Öffnung (21) der zweiten Blockiermittel umfaßt.

7. Nadelvorrichtung nach einem der Ansprüche 1 bis 6, dadurch gekennzeichnet, daß sie Mittel (7) zum Erleichtern der Einführung des vorspringenden Elements (4; 16) in den ersten Schlitz (6) am Ende des Schlitzes (6) umfaßt, das auf der Seite der Nadel (1) angeordnet ist.

8. Nadelvorrichtung nach Anspruch 6 oder 7, dadurch gekennzeichnet, daß die Mittel zum Erleichtern der Einführung des vorspringenden Elements (4; 16) in den Schlitz (6; 19) durch einen V-förmigen Ausschnitt (7; 10; 22) gebildet sind, der die Ränder des Schlitzes (6; 19) erweitert.

9. Nadelvorrichtung nach einem der Ansprüche 1 bis 8, dadurch gekennzeichnet, daß das vorspringende Element durch aneinandergeklappte Flügel (16) eines Schmetterlingsflügel-Greiforgans (15, 16, 17) gebildet ist, das gemeinsam mit einem Verbindungselement (3) ausgebildet ist, das die Basis der Nadel (1) mit dem Rohr (2) verbindet.
